# EUROPEAN PATENT APPLICATION

(11) **EP 2 019 091 A1**
(43) Date of publication of application: **28.01.2009**
(21) Application number: 07110956.5
(22) Date of filing: 25.06.2007
(51) Int. Cl.: C07C 69/94, C07D 213/79

(54) **Novel polyhydroxylated compounds as Fatty Acid Synthase (FASN) inhibitors**

(71) Applicant: Fundació Privada Institut d'Investigació Biomédica de Girona - Dr. Josep Trueta, 17007 Girona (ES); Universidad Complutense de Madrid, 28040 Madrid (ES)
(72) Inventor: Colomer Bosch, Ramón, 17007 Girona (ES); Puig Miquel, Teresa, 17007 Girona (ES); Brunet Vidal, Joan, 17007 Girona (ES); López Rodrígues, María, Luz, 28040 Madrid (ES); Benhamù Salama, Bellinda, 28100 Alcobendas (Madrid) (ES); Ortega Gutiérrez, Silvia, 28019 Madrid (ES); Turrado García, Carlos, 28925 Alcorcón (Madrid) (ES)
(74) Representative: Barlocci, Anna

(57) **Abstract**

The present invention relates to new polyhydroxylated compounds and, in particular, to its activity as fatty acid synthase (FASN) inhibitors and to their use for the treatment of pathological states for which an inhibitor of this enzyme is indicated. The invention further relate to pharmaceutical compositions containing them and to a process for the preparation of such compounds.

## Description

### FIELD OF THE INVENTION

The present invention relates to polyhydroxylated compounds and, in particular, to its activity as fatty acid synthase (FASN) inhibitors and to their use for the treatment of pathological states for which an inhibitor of this enzyme is indicated. The invention further relate to pharmaceutical compositions containing them and to a process for the preparation of such compounds.

### BACKGROUND ART

Fatty acid synthase (E.C. 2.3.1.85, FASN) is the key lipogenic enzyme required for catalysing de novo synthesis of long-chain fatty acids from acetyl-CoA, malonyl-CoA and NADPH precursors (Wakil, S.J. et al., Biochemistry 1989, 28, 4523-30). Normal cells preferentially use circulating dietary fatty acids for the synthesis of new structural lipids. Thus, FASN expression is generally low to undetectable in normal human tissues, other than the liver and adipose tissue. In contrast, high levels of FASN expresssion have been observed in several cancers, including breast, prostate, colon, ovary, endometrium, mesothelium, lung, thyroid, stomach and brain (reviewed by Kuhajda, F.P. et al., Cancer Res. 2006, 66, 5977-80). The widespread expression of FASN in human cancer and its association with poor prognosis suggest that fatty acid synthesis provides an advantage for tumor growth and could be a promising target for anti-tumor drug development.

The pharmacological inhibition of FASN has been used to study the loss of FASN function in tumor cells. Considerable interest has been developed in identifying novel inhibitors of FASN. The first identified was cerulenin, a natural antibiotic product of the fungus *Cephalosporium ceruleans* (Omura et al., Bacteriol. Rev. 1976, 40, 681-97). It has been reported that FASN inhibition by cerulenin leads to apoptotic cancer cell death (Menéndez, J.A. et al., Proc. Nat. Acad. Sci. 2004, 101, 10715-20). However, cerulenin's unstability renders it inappropriate as an *in vivo* anti-tumor agent. Compound C75 ((2R,3S)-4-methylene-2-octyl-5-oxotetrahydrofuran-3-carboxylic acid), a synthetic derivative related to cerulenin, having higher stability, has been tested recently for its anti-tumor effects (Kuhajda, F.P. et al., Proc. Nat. Acad. Sci. 2000, 97, 3450-4). FASN inhibition using C75 is cytotoxic for various tumor cell lines *in vitro,* and also shows growth inhibitory effects on cancer cell xenografts and transgenic mice *in vivo.* However, the use of C75 as an *in vivo* anti-tumor agent is limited by the dose-dependent anorexia that appears to be associated with the stimulation of carnitine palmitoyltransferase-1 (CPT-1) β-oxidation (Nicot, C. et al., Biochem. Biophys. Res. Comm. 2004, 325, 2745-7). It has been postulated that high levels of malonyl-CoA and CPT-1 inhibition might represent a mechanism whereby FASN inhibition leads to tumor cell death. Another novel FASN inhibitor with *in vivo* antitumor activity is the beta-lactone Orlistat (Kridel et al., Cancer Res. 2004, 64, 2070-5), an FDA-approved drug used for treating obesity. However, Orlistat possesses an extremely low oral bioavailability, so a novel formulation will be required for treating tumors.

Recently it has been reported that (-)-epigallocatechin-3-gallate (EGCG), the main polyphenolic catechin of green tea, and other naturally occurring flavonoids (luteolin, quercetin and kaempferol) inhibit FASN, induce apoptosis of several tumor cell lines *in vitro* and reduce the sizes of tumors in animal models (Brusselmans, K. et al., J. Biol. Chem. 2005, 280, 5636-45). We have recently reported that EGCG achieves all cellular, functional and molecular anti-tumor effects exhibited by C75, but it does not modulate CPT-1 activity. Thus EGCG probably avoids the weight-loss pathways *in vivo* that have been associated to C75 administration. However, the effectiveness of EGCG *in vivo* is limited due to its high IC₅₀ value (the inhibitory ability of EGCG is 4 fold lower than that of C75 and cerulenin) in cancer cells overexpressing FASN, which apparently would require high doses for *in vivo* administration. Furthermore, EGCG shows poor bioavailability as proved by Nakagawa K. (Nakagawa, K et al., Anal. Biochem. 1997, 248, 41-9). In this study it is shown that only 0.012% of EGCG is absorbed in rats after an oral dose of 56 mg of EGCG. This low absorption was attributed to the poor stability of EGCG in neutral or alkaline solutions. As pH value of the intestine and body fluid is neutral or slightly alkaline, green tea catechins will be unstable inside the human body, thus leading to reduced bioavailability.

It is also known that the modulation of fatty acid metabolism can be exploited to inhibit food intake. C75, originally designed as a FASN inhibitor, causes profound and reversible weight loss in lean mice, diet-induced obese (DIO) mice, leptin-deficient (ob/ob) mice and normal lean rats, resulting in loss of up to 12% of body mass within 24 h in the mice models (Kuhajda, F. et al., Trends Pharmacol. Sci. 2005, 26, 541-4). EP0869784-B1 relates to the use of FASN inhibitors, structurally unrelated to those herein described, to achieve weight loss and/or reduction of adipocyte mass without significant toxicity.

FASN inhibitors have been disclosed as agents for inducing weight loss and for inhibiting the growth of pre-existing cancer cells. For example, EP0869784-A discloses a method for inducing weight loss by the administration of a class of FASN inhibitors (γ-substituted-α-methylene-β-carboxy- γ-butyrolactone compounds). The EP0869784 patent also discloses that these compounds are useful for inhibiting the growth of pre-existing cancer cells.

EP651636-A discloses a method for treating pre-existing cancer by administering a FASN inhibitor at a dose that is selectively cytotoxic to cancer cells, but not to other types of non-transformed (normal) cells.

In summary, there is strong evidence indicating that FASN inhibitors are useful as agents for inducing weight loss and for inhibiting the growth of pre-existing cancer cells. Thus, the discovery of new FASN inhibitors is interesting in the therapy of these disorders or illnesses.

### SUMMARY OF THE INVENTION

Inventors have provided a series of novel polyhydroxylated compounds, which show significant inhibition of FASN without parallel stimulation of CPT-1 activity. As it is shown in the Examples, their effects in growth and signaling pathways in cancer cells have been tested. Cellular proliferation, fatty acid metabolism pathways (FASN and CPT-1 activities), induction of apoptosis (as assessed by cleavage of poly(ADP-ribose) polymerase (PARP) and cell signalling (HER2, ERK1/2 and AKT cascades) have been evaluated. The results show that the compounds of the invention are FASN inhibitors, indicating that these compounds are useful as a treatment for inducing weight loss and for inhibiting the growth of pre-existing cancer cells in mammals, including humans.

Therefore, according to the first aspect of the present invention, it provides polyhydroxylated compounds of formula (I), their stereoisomers, crystalline forms, prodrugs, pharmaceutically acceptable salts and pharmaceutically acceptable solvates: 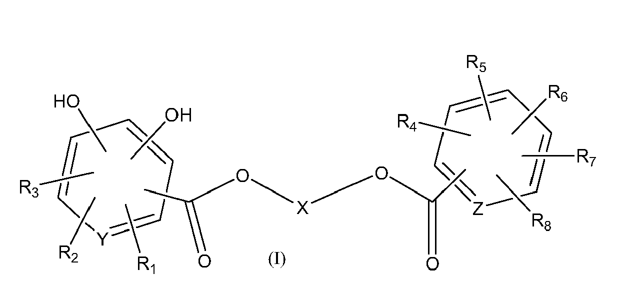 wherein:
R₁, R₂, R₃, R₄, R₅, R₆, R₇ and R₈ are independently selected from the group consisting of H, (C₁-C₄)-alkyl, (C₁-C₄)-alkoxy, halogen, nitro, NH₂, (C₁-C₄)alkyl ester, CONH₂, (C₁-C₄)alkylamide, CF₃, and hydroxyl;
Y and Z are each independently selected from C and N;
X is a biradical derived from an aromatic monoheterocycle being at least one of the members of the ring independently selected from N, O, and S; or a biradical derived from one of the known ring systems containing 2-3 rings of 5-6 members each ring, being the rings aromatic or partially unsaturated, isolated or fused; each ring optionally substituted with one or more groups selected from the group consisting of (C₁-C₄)-alkyl, and (C₁-C₄)-alkoxy, halogen, nitro, NH₂, (C₁-C₄)alkyl ester, CONH₂ and (C₁-C₄)alkylamide; being each one of the members of the ring independently selected from C, N, O and S;
with the proviso that X is not chromane, anthracene or a biradical of formula:

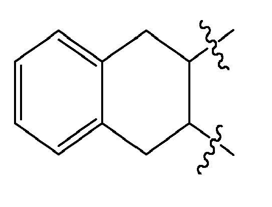

A second aspect of the invention relates to a pharmaceutical composition comprising an effective amount of a compound of formula (I), their stereoisomers, crystalline forms, prodrugs, pharmaceutically acceptable salts or pharmaceutically acceptable solvates, with the proviso that X is not chromane or 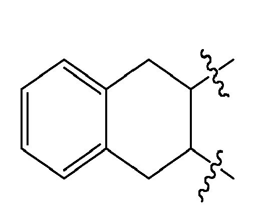 in combination with appropriate amounts of pharmaceutically acceptable diluents or carriers.

The present invention also relates to a pharmaceutical composition as defined above for the manufacture of a medicament for the treatment and/or prophylaxis of a very wide range of disorders mediated by FASN and associated clinical symptoms in mammals, including humans.

Accordingly, a third aspect of the invention relates to use of a compound of formula (I), as defined above, with the proviso that X is not chromane or 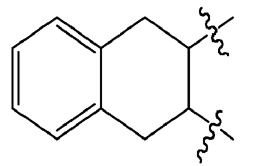 their stereoisomers, crystalline forms, prodrugs, pharmaceutically acceptable salts and pharmaceutically acceptable solvates as described herein, in the manufacture of a medicament for the treatment and/or prophylaxis of a disorder mediated by FASN and associated clinical symptoms in a mammal, including a human. Preferably such FASN mediated disorder is cancer or obesity.

This third aspect may alternatively be formulated as a method of treatment and/or prophylaxis of a mammal, including a human, suffering from or being susceptible to any of the diseases mentioned above which comprises administering to said patient a therapeutically effective amount of a compound of formula (**I**), with the proviso that X is not chromane or 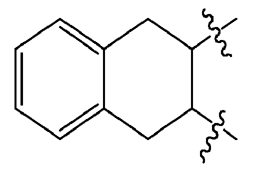 in combination with appropriate amounts of pharmaceutically acceptable diluents or carriers.

A fourth aspect of the invention relates to the process for the preparation of the compounds of formula (I) as defined herein, with the proviso that X is not chromane, anthracene or 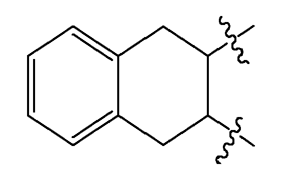 which comprises the following steps:
a) reacting a dihydroxyderivative of formula (**II**) 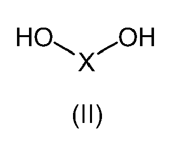 wherein X is as defined above;
   with carboxylic acids of formula **(III)** and **(IV)** 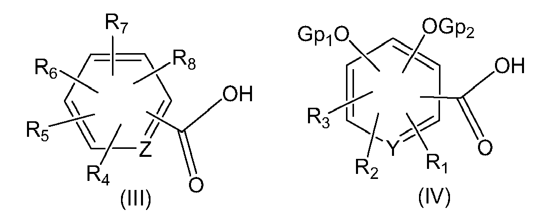 wherein Y, Z and R₁-R₈ are as defined above; and Gp₁ and Gp₂ are each independently a hydroxyl protecting group;
   resulting in protected intermediate of formula (V); and
b) deprotecting intermediate (V) 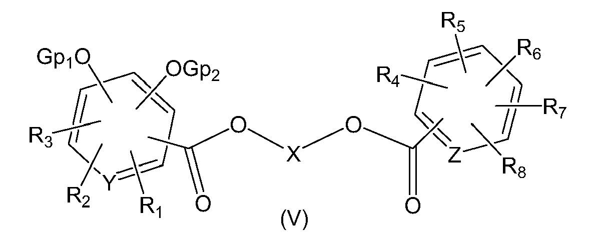 wherein Gp₁, Gp₂ and R₁-R₈ are as defined above;
   resulting in final compounds of formula **(I).**

Optionally, once the compound is obtained, it can be transformed into its pharmaceutically acceptable salt by conventional processes known in the art.

Optionally, once the compound or the pharmaceutically acceptable salt thereof is obtained, it can be transformed into a prodrug thereof using methods known in the art. For example the inventive compounds can be transformed into prodrugs by converting one or more of the hydroxy groups into esters.

Compounds of formula (I) wherein X is chromane, anthracene or 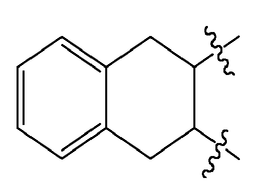 can be prepared analogously.

Both steps (a) and (b) of the process are illustrated in Scheme 1.

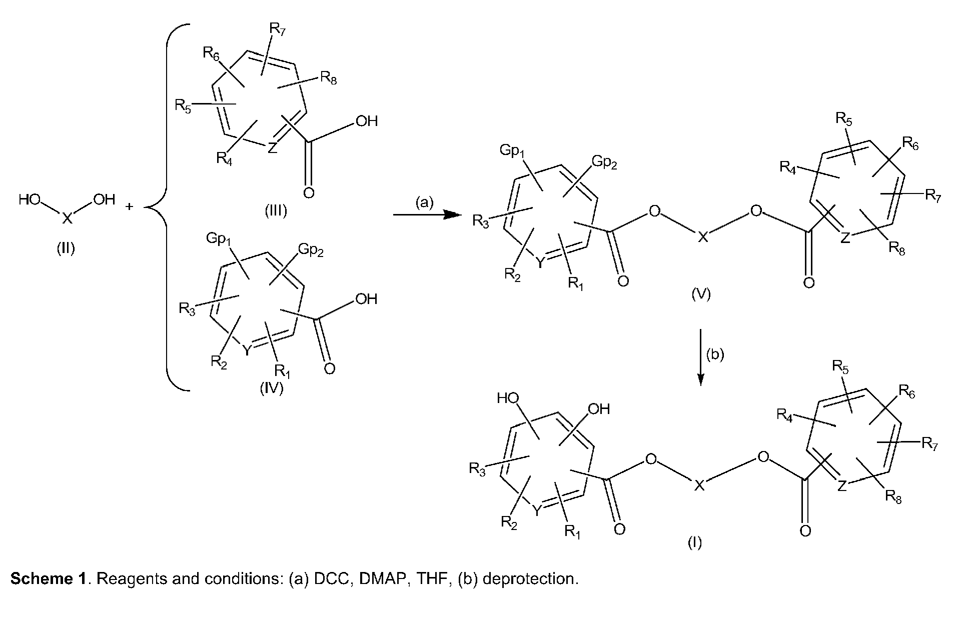

The invention additionally provides kits comprising separate containers containing a pharmaceutical composition as defined above, a reconstituting agent and instructions for the use of each actor in combination for the treatment or prevention of cancer. Methods of reconstitution are also provided.

### DETAILED DESCRIPTION OF THE INVENTION:

The inventors have surprisingly identified a class of compounds which shows a remarkable inhibitory FASN activity.

### Definitions

Prior to a discussion of the detailed embodiments of the invention a definition of specific terms related to the main aspects of the invention is provided.

The wavy line shows the attach point of the moiety.

The term "pharmaceutically acceptable salt", as used herein, refers to salts derived from organic and inorganic acids. The compound of the general formula (I) may be converted into its pharmaceutically acceptable salts, or its pharmaceutically acceptable solvates by conventional methods. For example, such salts may be prepared by treating one or more of the compounds with an aqueous solution of the desired pharmaceutically acceptable metallic hydroxide or other metallic base and evaporating the resulting solution to dryness, preferably under reduced pressure in a nitrogen atmosphere. Alternatively, a solution of the compound of formula (I) may be mixed with an alkoxide of the desired metal, and the solution subsequently evaporated to dryness. The pharmaceutically acceptable hydroxides, bases, and alloxides include those worth cations for this purpose, including (but not limited to), potassium, sodium, ammonium, calcium, and magnesium. Other representative pharmaceutically acceptable salts include hydrochloride, hydrobromide, sulphate, bisulphate, lactate, phosphate, tosylate, citrate, maleate, fumarate, succinate, tartrate, acetate, oxalate, propionate, nitrate, methanesulfonate, benzoate and similarly known acceptable acids. However, it will be appreciated that non-pharmaceutically acceptable salts also fall within the scope of the invention since those may be useful in the preparation of pharmaceutically acceptable salts.

The term "(C₁-C₄)-alkyl" as used herein refers to a saturated branched or linear hydrocarbon chain with 1 to 4 hydrocarbon atoms. Preferably "(C₁-C₄)-alkyl" is an unsubstituted group selected from methyl, ethyl, propyl, isopropyl, butyl, isobutyl, s-butyl and t-butyl.

The term "(C₁-C₄)-alkoxy" as used herein refers to a saturated branched or linear hydrocarbon chain with 1 to 4 hydrocarbon atoms (i.e. (C₁-C₄)-alkyl groups as defined above) linked to an oxygen, thus (C₁-C₄)-alkyl-O. Preferably "(C₁-C₄)-alkoxy" is an unsubstituted group selected from methoxy, ethoxy, propoxy, isopropoxy, butoxy, isobutoxy, s-butoxy, and t-butoxy.

The term "halogen" is meant to include fluorine, chlorine, bromine and iodine.

"Solvate" means a physical association of a compound of this invention with one or more solvent molecules. This physical association includes hydrogen bonding. In certain instances the solvate will be capable of isolation, for example when one or more solvent molecules are incorporated in the crystal lattice of the crystalline solid. "Solvate" encompasses both solution-phase and isolable solvates. Representative solvates include hydrates, ethanolates, methanolates, and the like.

The term "protecting group" refers to a chemical moiety or group which protects or prevents an active moiety or group from participating with or interfering with one or more chemical synthetic steps and its removal restores the moiety to its original active state. The term protecting group as used herein refers to those groups intended to protect against undesirable reactions during synthetic procedures. Such protecting groups are well known to those skilled in the art. Examples of protecting groups can be found in Green et al.,"Protective Groups in Organic Chemistry" (Wiley, 2nd ed. 1991), McOmie et al. "Protective Groups in Organic Chemistry" (Plenam Press, New York, 1973), and Harrison et al, "Compendium of Synthetic Organic Methods", Vols. 1-8 (John Wiley and Sons, 1971-1996). Protecting groups can be removed with inter alia acid, base, fluoride ions, hydrogenation, metals such as zinc as well as by numerous other methods which are well known in the art. One of ordinary skill in the art can readily choose an appropriate protecting group to facilitate synthetic reactions according to methodological aspects of the present invention without engaging in undue experimentation. Representative amino protecting groups include, but are not limited to, formyl, acetyl, trifluoroacetyl, benzyl, benzyloxycarbonyl ("CBz"), tert-butoxycarbonyl ("BOC"), trimethylsilyl ("TMS"), 2-trimethylsilyl-ethanesulfonyl ("SES"), trityl and substituted trityl groups, allyloxycarbonyl, 9-fluorenylmethyloxycarbonyl ("FMOC"), nitro-veratryloxycarbonyl ("NVOC") and the like. Representative hydroxy protecting groups include, but are not limited to, those where the hydroxy group is either acylated or alkylated such as benzyl (Bn), and trityl ethers as well as alkyl ethers, tetrahydropyranyl ethers, trialkylsilyl ethers and allyl ethers. Bn group or trialkyl silyl ethers are preferred groups for the protection of hydroxyl.

As used herein, the term "cancer development" is understood to mean the initial appearance of cancerous cells. By "cancerous cells" we mean cells which have the property of autonomous proliferation and have invaded adjacent tissues.

As used herein, the term "subject in need thereof" is understood to include subjects who have been diagnosed as pre-cancerous, have develop a cancer, or who may have a predisposition to develop the disease, genetically or otherwise. Alternatively, it also includes subjects who have been diagnosed as an obese patient, or who may have a predisposition to develop the disease, genetically or otherwise, wherein a weight loss is or may be indicated.

As used herein, the term "inhibiting" is understood to mean preventing, suppressing, retarding, blocking or delaying cancer development, such as, for example, by stimulating, inducing or triggering apoptosis (i. e., programmed cell death) in pre-cancerous cells.

The phrase "inhibiting the activity of FASN" as used herein refers to 10% to 100% decrease in FASN activity. More preferably, the term "inhibiting the activity of FASN" refers to 25% to 100% decrease in FASN activity, and most preferably, to 50% to 100% decrease in FASN activity. The invention contemplates the inhibition of FASN via any of the aforementioned seven enzymatic steps required for FASN activity and any inherent steps or processes. A decrease or change in FASN activity can be measured by any method known to one skilled in the art.

"Inhibitors of FASN" include competitive and non-competitive FASN inhibitors. A competitive FASN inhibitor is a molecule that binds the FASN enzyme in a manner that is mutually exclusive of substrate binding. Typically, a competitive inhibitor of FASN will bind to the active site. A non- competitive FASN inhibitor can be one which inhibits the synthesis of fatty acids, but its binding to the enzyme is not mutually exclusive over substrate binding. FASN inhibitors contemplated by this invention are compounds that reduce the activity of FASN in animal cells without any significant effect on other cellular activities, at least at comparable concentrations.

It is clear to a person skilled in the art that the compounds of the present invention may have at least one chiral center and thus form "stereoisomers", such as e.g. diastereomers. The racemic forms as well as all optical isomers are part of the present invention and are thus encompassed by the scope of the claims.

According to an embodiment of the invention, it provides polyhydroxylated compounds of formula (I) wherein X is a biradical derivative of an aromatic monoheterocycle, being at least one of the members of the ring independently selected from N, O, and S, or a biradical derivative of one of the known ring systems containing 2-3 rings of 5-6 members each ring, being the rings aromatic or partially unsaturated, isolated or fused; each optionally substituted with one or more groups selected from the group consisting of (C₁-C₄)-alkyl, and (C₁-C₄)-alkoxy, halogen, nitro and amine, ester, amide; being each one of the members of the ring independently selected from C and N;
with the proviso that X is not anthracene or

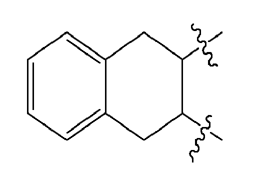

According to a preferred embodiment of the invention, the compounds of formula (I), are those wherein X is selected from the group consisting of naphthalene, biphenyl optionally substituted and pyridine.

When X is a biphenyl, such biphenyl moiety can be selected from:

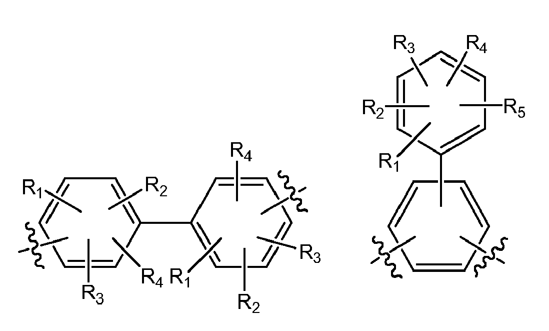

More preferred are those wherein X is selected from the group consisting of

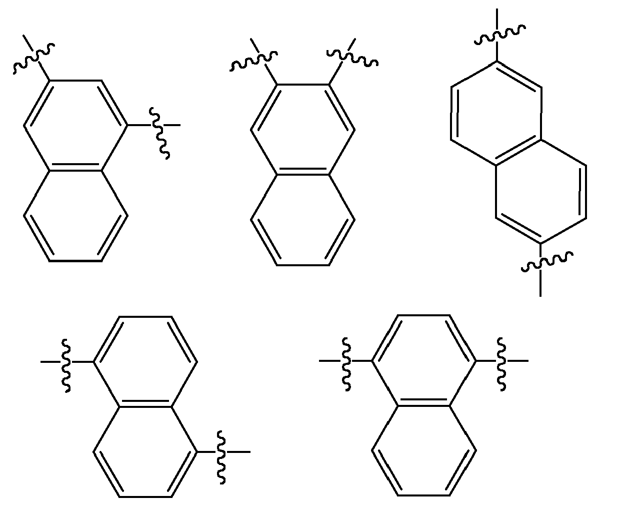

The following compounds are particularly preferred:
2,3-bis[(3,4,5-trihydroxybenzoyl)oxy]naphthalene (e);
1,3-bis[(3,4,5-trihydroxybenzoyl)oxy]naphthalene (f);
1,3-bis[(3,5-dihydroxybenzoyl)oxy]naphthalene (g);
1,3-bis[(3,4-dihydroxybenzoyl)oxy]naphthalene (h);
1,4-bis[(3,4,5-trihydroxybenzoyl)oxy]naphthalene (i);
1,4-bis[(3,5-dihydroxybenzoyl)oxy]naphthalene (j);
1,4-bis[(3,4-dihydroxybenzoyl)oxy]naphthalene (k);
2,6-bis[(3,4,5-trihydroxybenzoyl)oxy]naphthalene (l);
1,5-bis[(3,4,5-trihydroxybenzoyl)oxy]naphthalene (m);
2,5-bis[(3,4,5-trihydroxybenzoyl)oxy]-1,1'-biphenyl (o);
6-(benzoyloxy)-1,1'-biphenyl-3-yl 3,4,5-trihydroxybenzoate (p);
4,4'-bis[(3,4,5-trihydroxybenzoyl)oxy]-1,1'-biphenyl (q); and
4,4'-bis[(2,6-dihydroxyisonicotinoyl)oxy]-1,1'-biphenyl (r).

The final products have been structurally characterized by IR, NMR and MS techniques. For greater ease of handling, when the final product is not crystalline, it is transformed in a pharmaceutically acceptable salt, derived from an inorganic or organic acid or base.

The compounds of the invention showed significant inhibition of FASN.

As it is shown in the Examples, compounds **f** and **i** potently inhibited FASN activity (reduction of 50% of FASN activity after 6 h of cell exposure), without parallel stimulation of CPT-1 activity. In SK-Br3 cells, the relative IC₅₀ ratio of EGCG to compound **f** is 7.5 (150 µM/20µM) and to compound **i** is 5.4 (150 µM/28 µM). Compounds **f** and **i** induced apoptosis (cleavage of PARP) and caused a marked decrease in the active forms of oncoprotein HER2, Akt and ERK1/2 within 2 h after treatment. At similar inhibitor concentrations, non-malignant cells were not affected. Taken together, the results show that compounds **f** and i are potent and specific inhibitors of FASN and inhibit the growth of breast cancer cells.

In the field of drug design, any structural modification of a pharmacologically active compound was, in the absence of an established correlation between structural features and activity, expected a priori to disturb the pharmacological activity profile of the initial structure.

The compounds of the invention are structurally different from the compounds described in the prior art because of the novel combination of substituents of the formula, and particularly in the specific selection of central moiety (X) present in their structure. These structural variations are neither disclosed nor suggested in the prior art. These structural variations result in compounds that are useful as FASN inhibitors, with a remarkable activity inhibiting the growth of different cancer cell lines.

Any compound that is a prodrug of a compound of formula (I) as defined herein is within the scope and spirit of the invention. The term "prodrug" is used in its broadest sense and encompasses those derivatives that are converted in vivo by metabolic means (e.g., by hydrolysis) to the compounds of the invention, including N-oxides thereof. Prodrugs are often useful because, in some situations, they may be easier to administer than the parent drug. They may, for instance, be bioavailable by oral administration whereas the parent drug is not. The prodrug may also have improved solubility in pharmaceutical compositions over the parent drug. The prodrug may have improved chemical stability, improved patient acceptance and compliance, improved bioavailability, prolonged duration of action, improved organ selectivity, improved formulation (e.g. increased hydrosolubility), and/or decreased side effects (e.g. toxicity).

For more information on the type of prodrug envisaged in the present invention, the following references can be mentioned: Fleicher et al., Advanced Drug Delivery Review 19 (1996) 115-130; Design of prodrugs, H. Bundgaard, Ed., Elsevier, 1985; H. Bungaard, Drugs of the Future 16 (1991) 443; Saulnier et al. Bioorg. Med. Chem. Lett. 4 (1994) 1985; Safadi et al. Pharmaceutical Res. 10 (1993) 1350. Among the different appropriate prodrugs of the compounds of formula (I) there can preferably be in the form of esters of the hydroxy groups.

### Pharmaceutical Product

In other embodiments, the invention provides pharmaceutical compositions containing one or more of the compounds of formula (I), their stereoisomers, prodrugs, pharmaceutically acceptable salts or pharmaceutically acceptable solvates, and optionally one or more pharmaceutically acceptable carriers, excipients or diluents. The term "carrier", as used herein, shall encompass carriers, excipients and diluents.

Examples of such carriers are well known to those skilled in the art and are prepared in accordance with acceptable pharmaceutical procedures. Pharmaceutically acceptable carriers are those carriers that are compatible with the other ingredients in the formulation and are biologically acceptable.

Administration of the compounds or compositions of the present invention may be by any suitable method, such as orally, transdermally, parenterally, intramuscularly, intravenously, subcutaneously or by other modes of administration. Preferably, the pharmaceutical product can be administered orally. Preferably pharmaceutical compositions of the compounds of the invention include liquid (solutions, suspensions or emulsions) with suitable composition for intravenous administration, and they may contain the pure compound or in combination with any carrier or other pharmacologically active compounds.

We prefer that infusion times of up to 24 hours are used, more preferably 2-12 hours, with 2-6 hours most preferred. Short infusion times which allow treatment to be carried out without an overnight stay in hospital are especially desirable. However, infusion may be 12 to 24 hours or even longer if required. Infusion may be carried out at suitable intervals of say 1 to 4 weeks. The administration can be performed in cycles, in a preferred application method, an intravenous infusion of a compound of the invention is given to the patients the first week of each cycle, and the patients are allowed to recover for the remainder of the cycle. The preferred duration of each cycle is of either 1, 3 or 4 weeks; multiple cycles can be given as needed. Other protocols can be devised as variations. Dose delays and/or dose reductions and schedule adjustments are performed as needed depending on individual patient tolerance of treatments. Although guidance for the dosage is given above, the correct dosage of the compound will vary according to the particular formulation, the mode of application, and the particular situs, host and tumour being treated. Other factors like age, body weight, sex, diet, time of administration, rate of excretion, condition of the host, drug combinations, reaction sensitivities and severity of the disease shall be taken into account. Administration can be carried out continuously or periodically within the maximum tolerated dose.

Pharmaceutical compositions containing compounds of the invention may be delivered by liposome or nanosphere encapsulation, in sustained release formulations or by other standard delivery means.

The correct dosage of the compounds will vary according to the particular formulation, the mode of application, and the particular situs, host and tumour being treated.

The compounds and compositions of this invention may be used with other drugs to provide a combination therapy. The other drugs may form part of the same composition, or be provided as a separate composition for administration at the same time or at different time.

Representative solid carriers include one or more substance that can act as flavouring agents, lubricants, solubilizers, suspending agents, fillers, glidants, compression aids, binders, tablet-disintegrating agents, or encapsulating materials. Oral formulations containing the active compounds of this invention may comprise any conventionally used oral forms, including tablets, capsules, buccal forms, troches, lozenges and oral liquids, suspensions or solutions. In powders, the carrier is a finely divided solid that is in admixture with the finely divided active ingredient. In tablets, the active ingredient is mixed with a carrier having the necessary compression properties in suitable proportion and compacted in the shape and size desired.

Capsules may contain mixtures of the active compound(s) with inert fillers and/or diluents such as the pharmaceutically acceptable starches, sugars, artificial sweetening agents, powdered celluloses, such as crystalline and microcrystalline celluloses, flours, gelatins, gums, etc.

Useful tablet formulations may be made by conventional compression, wet granulation or dry granulation methods and utilize pharmaceutically acceptable diluents, binding agents, lubricants, disintegrants, surface modifying agents (including surfactants), suspending or stabilizing agents, including, but not limited to, magnesium stearate, stearic acid, sodium lauryl sulfate, microcrystalline cellulose, methyl cellulose, sodium carboxymethyl cellulose, carboxymethylcellulose calcium, polyvinylpyrrolidine, gelatin, alginic acid, acacia gum, xanthan gum, sodium citrate, complex silicates, calcium carbonate, glycine, dextrin, sucrose, sorbitol, dicalcium phosphate, calcium sulfate, lactose, kaolin, mannitol, sodium chloride, talc, starches, sugars, low melting waxes, and ion exchange resins. Preferred surface modifying agents include nonionic and anionic surface modifying agents. Representative examples of surface modifying agents include, but are not limited to, poloxamer 188, benzalkonium, chloride, calcium stearate, cetostearyl alcohol, cetomacrogol emulsifying wax, sorbitan esters, colloidol silicon dioxide, phosphates, sodium dodecylsulfate, magnesium aluminium silicate, and triethanolarnine. Oral formulations herein may utilize standard delay or time release formulations to alter the absorption of the active compound(s). The oral formulation may also consist of administering the active ingredient in water or a fruit juice, containing appropriate solubilizers or emulsifiers as needed.

Liquid carriers can be used in preparing solutions, suspensions, emulsions, syrups, and elixirs. The active ingredient can be dissolved or suspended in a pharmaceutically acceptable oil or fat. The liquid carrier can contain other suitable pharmaceutical additives such as, for example, solubilizers, emulsifiers, buffers, preservatives, sweeteners, flavouring agents, suspending agents, thickening agents, colours, viscosity regulators, stabilizers or osmoregulators. Suitable examples of liquid carriers for oral and parenteral administration include water (particularly containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil).

For parenteral administration, the carrier can also be an oily ester such as ethyl oleate and isopropyl myristate. Sterile liquid carriers are used in sterile liquid form compositions for parenteral administration. The liquid carrier for pressurized compositions can be an halogenated hydrocarbon or other pharmaceutically acceptable propellant.

The compounds of this invention may also be administered parenterally or intraperitoneally. Solutions or suspensions of these active compounds as a free base or pharmacologically acceptable salt can be prepared in water suitably mixed with a surfactant such as hydroxypropylcellulose. Dispersions can also be prepared in glycerol, liquid polyethylene glycols and mixtures thereof in oils. Under ordinary conditions of storage and use, these preparations contain a preservative to inhibit the growth of microorganisms.

The pharmaceutical forms suitable for injectable use include sterile aqueous solutions or dispersions and sterile powders for the extemporaneous preparation of sterile injectable solutions or dispersions. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

Optionally, the compounds of the invention can be suitably prepared in a lyophilised form suited for reconstitution. Reconstitution is preferably effected with a mix of emulsifying solubiliser, alkanol and water. The lyophilised composition preferably comprises mainly the bulking agent, such as at least 90 % or at least 95 % bulking agent.

The formulations may be presented in uni-dose or multi-dose containers, for example sealed ampoules and vials, and may be stored in a freeze-dried (lyophilized) condition requiring only the addition of the sterile liquid carrier, for example water for injections, immediately prior to use. Thus the invention additionally provides kits comprising separate containers containing the lyophilised composition and the reconstituting agent. Methods of reconstitution are also provided.

A pharmaceutical product, as described herein, may include other pharmaceutically active substances. It can be prepared by mixing the active compounds with one or more pharmacologically tolerated carriers and converting the mixture into a suitable pharmaceutical form.

### Use in clinical symptoms

Taking into account its remarkable inhibition of FASN, the compounds of formula (I) are useful in the treatment and/or prophylaxis of pathological states wherein the inhibition of FASN are indicated, such as, for example, the treatment and/or prophylaxis of cancer or obesity in mammals, particularly in humans.

Cancers that can be treated using the compounds of the invention include, but are not limited to, blood cell cancers such as autoimmune lymphoproliferative syndrome (ALPS), chronic lymphoblastic leukemia, hairy cell leukemia, chronic lymphatic leukemia, peripheral T-cell lymphoma, small lymphocytic lymphoma, mantle cell lymphoma, follicular lymphoma, Burkitt's lymphoma, Epstein-Barr virus-positive T cell lymphoma, histiocytic lymphoma, Hodgkin's disease, diffuse aggressive lymphoma, acute lymphatic leukemia, T gamma lymphoproliferative disease, cutaneous B cell lymphoma, cutaneous T cell lymphoma (i.e., mycosis fungoides), Sezary syndrome, acute myelogenous leukemia, chronic or acute lymphoblastic leukemia and hairy cell leukemia. Additional cancers that can be treated by the methods of the invention include solid tumors, including sarcoma, osteosarcoma, and carcinoma, such as adenocarcinoma (for example, breast cancer) and squamous cell carcinoma, Epstein-Barr virus-positive nasopharyngeal carcinoma, glioma, colon, stomach, prostate, renal cell, cervical and ovarian cancers, lung cancer (small cell lung carcinoma (SCLC) and non-small cell lung carcinoma (NSCLC)). Prefered cancers are breast, prostate, colon, ovary, endometrium, mesothelium, lung, thyroid, stomach and brain.

Malignancies with invasive metastatic potential can also be treated with the methods of the invention, including multiple myeloma. By treatment of the above described cancers, it is contemplated that symptoms associated with cancer will be relieved or ameliorated, such as cancer-associated cachexia, fatigue, asthenia, paraneoplastic syndrome of cachexia and hypercalcemia.

In yet another embodiment, in cases where a patient is being treated for a solid tumor or a tumor that has metastasized, it is contemplated that the co-administration of the compound of the invention with an immune cell activator follows surgical reduction of the tumor mass. In addition, it is contemplated that the patient can be treated in an early stage in the disease progression so that the patient is not immunologically suppressed or exhausted.

A therapeutically effective amount refers to that amount of the compound sufficient to result in amelioration of symptoms. Symptoms of cancer include pain, wasting and/or loss of appetite, tumor burden, nausea, fatigue, diarrhea, vomiting, and constipation. When a FASN inhibitor is co-administered with another therapeutic agent, doses are modified according to any interactions that can occur between the therapeutic agents.

The compounds described herein can be used in the preparation of a medicament for the treatment of tumour cells expressing at least one enzyme of the fatty acid biosynthetic pathway. Particularly in the preparation of a medicament for the treatment of tumour cells expressing fatty acid synthase (FASN) and/or of a condition responsive to reduction in adipose tissue mass or for treatment to induce weight loss.

Throughout the description and claims the word "comprise" and variations of the word, such as "comprising", are not intended to exclude other technical features, additives, components, or steps.

Additional objects, advantages and features of the invention will become apparent to those skilled in the art upon examination of the description or may be learned by practice of the invention. The following examples and drawings are provided by way of illustration, and are not intended to limit the present invention.

### BRIEF DESCRIPTION OF THE DRAWINGS

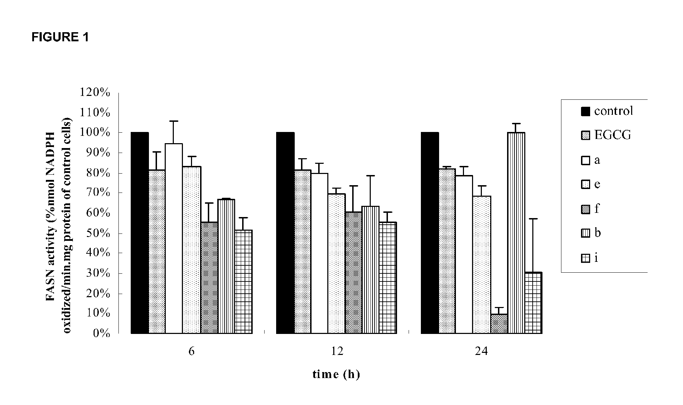

Figure 1. EGCG and novel compounds a, b, e, f and i inhibit FASN activity in SK-Br3 breast cancer cells. Cells were treated for 6, 12 and 24 hours with tested compounds and FASN activity (%nmol NADPH oxidized/min.mg protein of control cells) was assayed spectrophotometrically in particle-free supernatants.

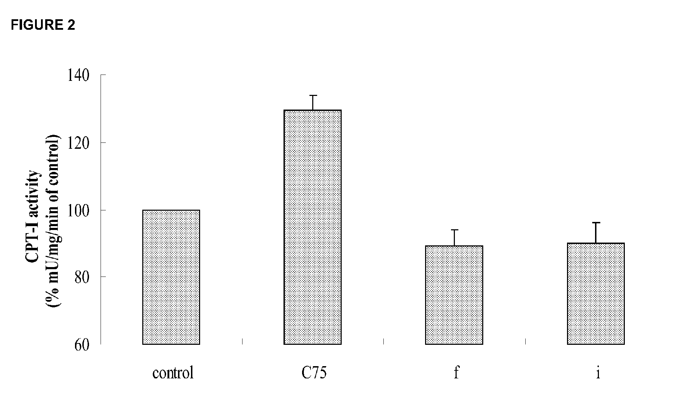

Figure 2. C75 stimulates CPT-1A activity, while compounds f and i did not. P. pastoris was transformed with the plasmid encoding for the rat CPT-1A. Mitochondria isolated from these cells were assayed for CPT-1A activity (% mU/mg/min of control) in the presence of DMSO (control), C75, compound f or compound i.

### DETAILED DESCRIPTION OF PARTICULAR EMBODIMENTS

### Example 1. Synthesis of compounds of general structure (I). General procedure. (See scheme 1)

A suspension of the carboxylic acid of formula (**IV**) (2.2 equiv.), DCC (2.2 equiv.) and DMAP (0.2 equiv.) in dry THF was stirred under an argon atmosphere at 0 °C for 15 min. Then, a solution of the dihydroxyderivative of formula **(II)** (1 equiv.) in THF was added dropwise and the reaction mixture was allowed to reach room temperature and stirred overnight. The mixture was filtered and the solvent was evaporated to dryness under reduced pressure. The crude was resuspended in dichloromethane and washed with a 5% aqueous solution of sodium bicarbonate. The organic layer was dried and after evaporation of the solvent the residue was purified by column chromatography in silica gel using the appropriate eluent, to afford the protected intermediate of general formula (**V**).

Alternatively, the carboxylic acid of formula (**III**) (1.1 equiv.) is treated with dihydroxyderivative of formula (**II**) (1 equiv.) using the same reagents and conditions, and the resultant monoester (1 equiv.) is subsequently coupled with the carboxylic acid of formula (**IV**) (1.1 equiv.) using the same reagents and conditions, to afford the protected intermediate of general formula (**V**).

To a solution of the benzylated intermediate of formula (**V**) in a mixture of dichloromethane/ethanol, 20% palladium hydroxide on carbon was added, and the mixture was hydrogenated at room temperature. Then, the catalyst was filtered and the solvents were evaporated under reduced pressure to afford the desired final compound of formula (I) as a solid, which was purified by recrystallization form the appropriate solvent.

Alternatively, a solution of the silylated intermediate of formula (**V**) in tetrahydrofuran or *N,N*-dimethylformamide was treated with hydrofluoric acid at room temperature. The precipitated solid was filtered, washed with dichloromethane, and the final compound of formula (I) was recrystallized from the appropriate solvent.

### Example 2. 1,2-bis[(3,4,5-trihydroxybenzoyl)oxy]benzene (a)

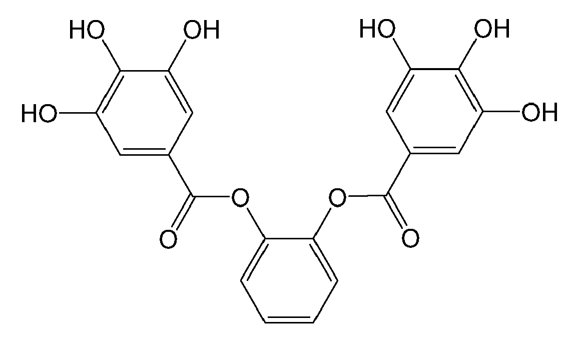

The title compound was prepared following general procedure described in example 1, starting from gallic acid and pyrocatechol as starting materials.

Yield: 37% (a), 44% (b); mp: 210 °C. IR (KBr): 3427, 1689, 1624 cm⁻¹. ¹H NMR (CD₃OD): δ 7.08 (s ,4H), 7.29-7.37 (m, 4H). ¹³C NMR (DMSO): δ 109.1 (4C), 118.1 (2C), 123.2 (2C), 126.0 (2C), 142.0 (4C), 145.0 (4C), 163.5 (2C). MS: M-H= 412.9.

### Example 3. 1,3-bis[(3,4,5-trihydroxybenzoyl)oxy]benzene (b)

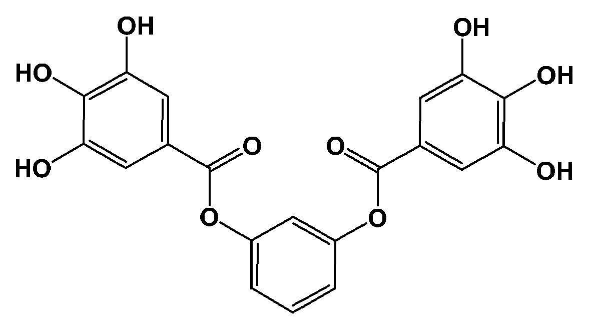

The title compound was prepared following general procedure described in example 1, starting from gallic acid and resorcinol as starting materials.

Yield: 68% (a), 38% (b); mp: 194-195 °C. IR (KBr): 3370, 1718, 1618, 1200 cm⁻¹. ¹H NMR (CD₃OD): δ 7.06-7.14 (m, 3H), 7.20 (s, 4H), 7.48 (t, *J* = 8.4, 1 H). ¹³C NMR (CD₃OD): δ 110.7 (4C), 117.1 (2C), 120.3 (2C), 130.8 (2C), 140.8 (2C); 146,8 (4C), 153.3 (2C), 166.7 (2C). MS: M + H = 412.8.

### Example 4. 2-methyl-1,4-bis[(3,4,5-trihydroxybenzoyl)oxy]benzene (c)

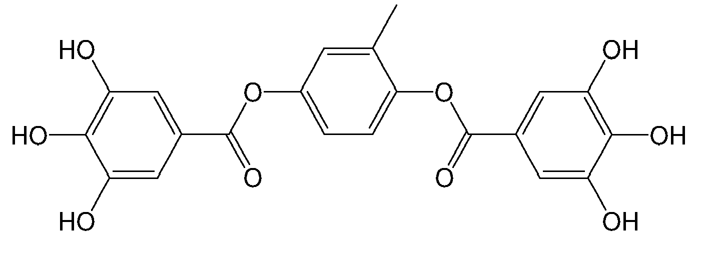

The title compound was prepared following general procedure described in example 1, starting from gallic acid and 2-methylbenzene-1,4-diol as starting materials.

Yield: 52% (a), 67% (b); mp: 256 °C (d). IR (KBr): 3385, 1692, 1604, 1209 cm⁻¹. ¹H NMR (CD₃OD): δ 2.31 (s, 3H), 6,95 (dd, J = 8.6, 2.9, 1 H), 7.01-7.05 (m, 2H), 7.10 (s, 2H), 7,13 (s, 2H). ¹³C NMR (CD₃OD): δ 16.4, 110.7 (4C), 120.4, 120.6, 121.3, 124.1, 125.2, 133.1, 140.6, 140.7, 146.7 (2C), 146.8 (2C), 148.0, 150.1, 166.7, 167.0. MS: M + H = 429.0.

### Example 5. 2-methoxy-1,4-bis[(3,4,5-trihydroxybenzoyl)oxy]benzene (d)

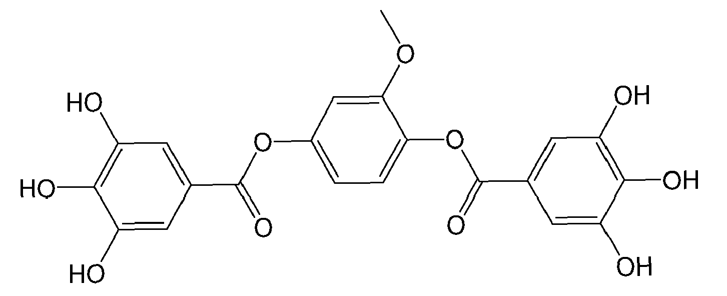

The title compound was prepared following general procedure described in example 1, starting from gallic acid and 2-methoxybenzene-1,4-diol as starting materials.

Yield: 45% (a), 77% (b); mp: 214-216 °C. IR (KBr): 3369, 1712, 1616, 1175 cm⁻¹. ¹H NMR (CD₃OD): δ 3.70 (s, 3H), 6.70 (dd, J = 8.6, 2.5, 1 H), 6.87 (d, J = 2.5, 1 H), 7.05 (d, J = 8.6, 1 H), 7.09 (s, 2H), 7.11 (s, 2H). ¹³C NMR (CD₃OD): δ 56.7, 108.2, 110.7 (2C), 110.8 (2C), 114.7, 120.4, 120.5, 124.2, 139.1 (2C), 140.7, 146.7 (2C), 146.8 (2C), 151.0, 153.5, 166.7, 167.0. MS: M + H = 444.9.

### Example 6. 2,3-bis[(3,4,5-trihydroxybenzoyl)oxy]naphthalene (e)

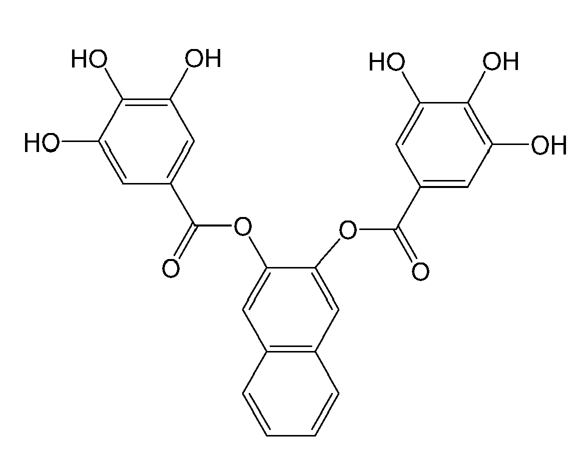

The title compound was prepared following general procedure described in example 1, starting from gallic acid and naphthalene-2,3-diol as starting materials.

Yield: 54% (a), 40% (b); mp: 182-183 °C. IR (KBr): 3308, 1743, 1618, 1194 cm⁻¹. ¹H NMR (CD₃OD): δ 7,12 (s, 4H), 7,51-7,56 (m, 2H), 7,79 (s, 2H), 7,89-7,92 (m, 2H). ¹³C NMR (CD₃0D): δ 109.3 (4C), 118.4 (2C), 120.6 (2C), 125.9 (2C), 126.9 (2C), 131.7 (2C), 139.2 (2C), 141.9 (2C), 145.1 (4C), 165.0 (2C). MS: M + Na= 487.0.

### Example 7. 1,3-bis[(3,4,5-trihydroxybenzoyl)oxy]naphthalene (f)

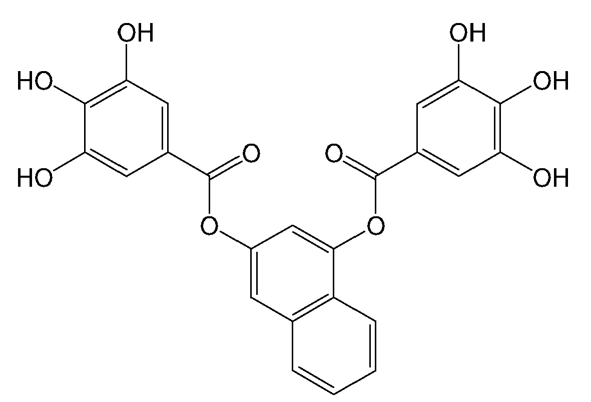

The title compound was prepared following general procedure described in example 1, starting from gallic acid and naphthalene-1,3-diol as starting materials.

Yield: 47% (a), 50% (b); mp: 163-164 °C. IR (KBr): 3385, 1709, 1608, 1198 cm⁻¹. ¹H NMR (CD₃OD): δ 7.18-7.27 (m, 3H), 7.34 (s, 2H), 7.45-7.65 (m, 3H), 7.88-7.96 (m, 2H). ¹³C NMR (CD₃OD): δ 109.7 (2C), 109.8 (2C), 114.7, 116.8, 119.1, 119.4, 121.4, 125.6, 126.3, 127.4, 128.0, 134.8, 139.7, 139.9, 145.8 (2C), 145.9 (2C), 148.1, 148.6, 165.7, 165.9. MS: M - H = 462.7.

### Example 8. 1,3-bis[(3,5-dihydroxybenzoyl)oxy]naphthalene (g)

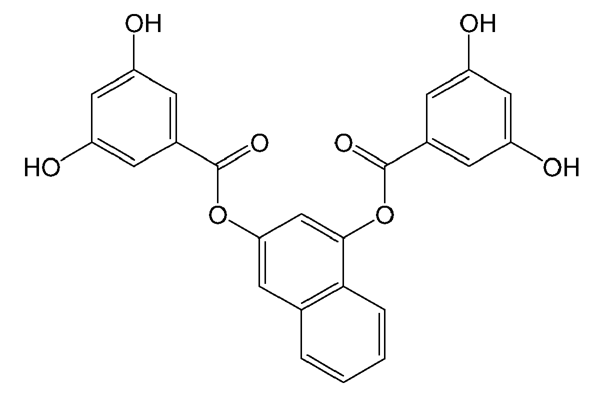

The title compound was prepared following general procedure described in example 1, starting from 3,5-dihydroxybenzoic acid and naphthalene-1 ,3-diol as starting materials.

Yield: 60% (a), 52% (b); mp: 167-169 °C (methanol/dichloromethane). IR (KBr): 3386, 1710, 1603, 1166 cm⁻¹. ¹H NMR (CD₃OD): δ 6.58 (t, J = 1.5, 1 H), 6.62 (t, J = 1.5, 1 H), 7.15 (d, J = 1.5, 2H), 7.23 (d, J = 1.5, 2H), 7.34 (d, J = 1.5, 1 H), 7.54-7.60 (m, 2H), 7.71 (d, J = 1.3, 1 H), 7.90-7.99 (m, 2H). ¹³C NMR (CD₃OD): δ 109.0 (2C), 109.3 (4C), 115.4, 117.9, 122.2, 126.4, 127.4, 128.4, 129.9, 131.7, 132.1, 135.7, 148.8, 149.3, 160.0 (2C), 160.1 (2C), 166.3, 166.5. MS: M + Na = 454.8.

### Example 9. 1,3-bis[(3,4-dihydroxybenzoyl)oxy]naphthalene (h)

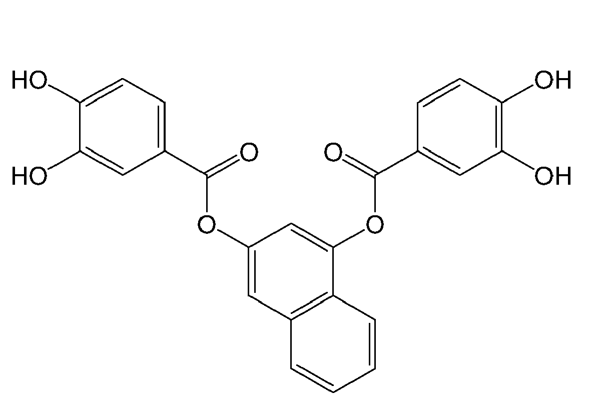

The title compound was prepared following general procedure described in example 1, starting from 3,4-dihydroxybenzoic acid and naphthalene-1 ,3-diol as starting materials.

Yield: 51% (a), 26% (b); mp: 79-81 °C (methanol/dichloromethane). IR (KBr): 3423, 1712, 1603, 1126 cm⁻¹. ¹H NMR (CD₃OD): δ 6.90 (d, *J* = 8.2, 1H), 6.95 (d, J = 8.2, 1 H), 7.29 (d, J = 2.1, 1H), 7.50-7.75 (m, 7H), 7.89-7.97 (m, 2H). ¹³C NMR (CD₃OD): δ 115.8, 116.2, 116.4, 117.9, 118.0 (2C), 121.3, 121.6, 122.4, 124.6, 124.7, 126.6, 127.4, 128.4, 129.0, 135.8, 146.5, 146.7, 149.1; 149.6, 152.7, 152.9, 166.6, 166.7. MS: M +Na = 454.9.

### Example 10. 1,4-bis[(3,4,5-trihydroxybenzoyl)oxy]naphthalene (i)

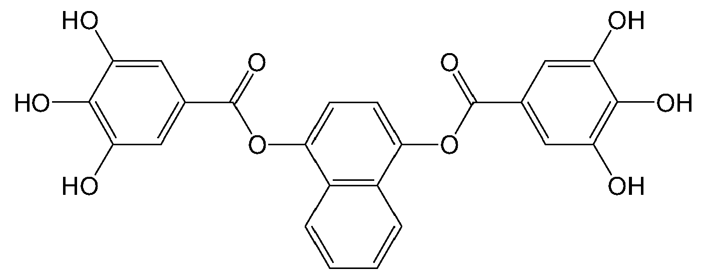

The title compound was prepared following general procedure described in example 1, starting from gallic acid and naphthalene-1,4-diol as starting materials.

Yield: 64% (a), 88% (b); mp: 252 °C (d). IR (KBr): 3406, 1718, 1612, 1196 cm⁻¹. ¹H NMR (CD₃OD): δ 7.36 (s, 4H), 7.39 (s, 2H), 7.57-7.60 (m, 2H), 7.92-7.95 (m, 2H). ¹³C NMR (CD₃OD): δ 110.8 (4C), 119.2 (2C), 120.2 (2C), 122.8 (2C), 128.2 (2C), 129.4 (2C), 140.9 (2C), 146.2 (2C), 146.9 (4C), 167.1 (2C). MS: M+Na=486.9.

### Example 11. 1,4-bis[(3,5-dihydroxybenzoyl)oxy]naphthalene (j)

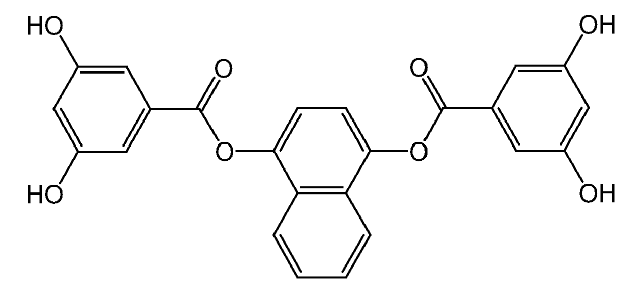

The title compound was prepared following general procedure described in example **1,** starting from 3,5-dihydroxybenzoic acid and naphthalene-1 ,4-diol as starting materials.

Yield: 57% (a), 60% (b); mp: 201-203 °C (methanol/dichloromethane). IR (KBr): 3406, 1701, 1608, 1155 cm⁻¹. ¹H NMR (CD₃OD): δ 6.63 (t, J = 2.2, 1H), 7.24 (d, J = 4.6, 2H), 7.42 (s, 2H), 7.59-7.62 (m, 2H), 7.93-7.96 (m, 2H). ¹³_{C} NMR (CD₃OD): δ 109.2 (2C), 109.3 (4C), 119.1 (2C), 122.6 (2C), 128.2 (2C), 129.1 (2C), 131.9 (2C), 146.0 (2C), 160.2 (4C), 166.9 (2C). MS: M + Na = 454.8.

### Example 12. 1,4-bis[(3,4-dihydroxybenzoyl)oxy]naphthalene (k)

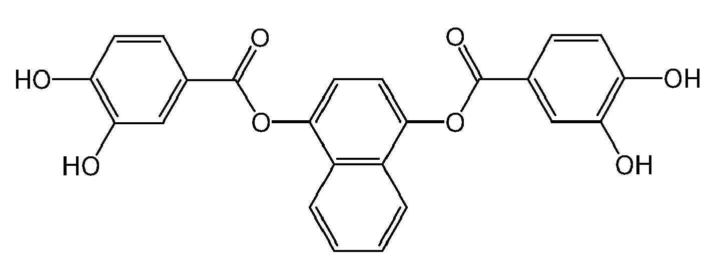

The title compound was prepared following general procedure described in example 1, starting from 3,4-dihydroxybenzoic acid and naphthalene-1 ,4-diol as starting materials.

Yield: 27% (a), 76% (b); mp: 261 °C (methanol/dichloromethane). IR (KBr): 3497, 1700, 1610, 1122 cm⁻¹. ¹H NMR (CD₃OD): δ 6.93-7.01 (m, 2H), 7.40 (s, 2H), 7.57-7.63 (m, 2H), 7,70-7.77 (m, 4H), 7.91-7.95 (m, 2H). ¹³C NMR (CD₃OD): δ 116.3 (2C), 118.0 (2C), 119.3 (2C), 121.5 (2C), 122.8 (2C), 124.7 (2C), 128.2 (2C), 129.4 (2C), 146.2 (2C), 146.7 (2C), 152.9 (2C), 166.9 (2C). MS: M + Na = 454.8.

### Example 13. 2,6-bis[(3,4,5-trihydroxybenzoyl)oxy]naphthalene (l)

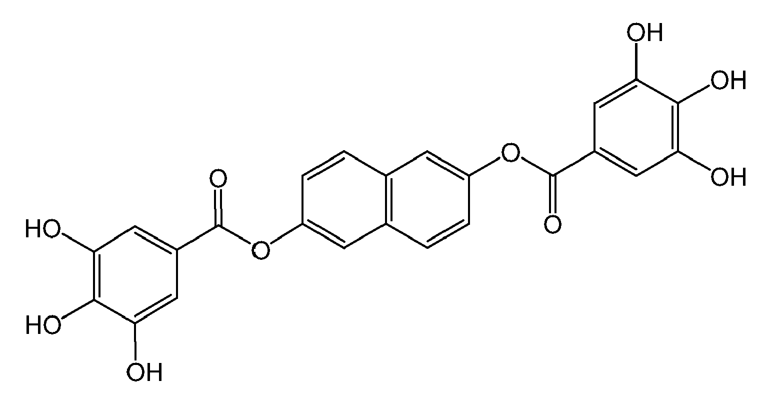

The title compound was prepared following general procedure described in example 1, starting from gallic acid and naphthalene-2,6-diol as starting materials.

Yield: 40% (a), 54% (b); mp: 272 °C (d). IR (KBr): 3415, 1712, 1608, 1205 cm⁻¹. ¹H NMR (CD₃OD): δ 7.21 (s, 4H), 7.32 (dd, J = 8.8, 2,2, 2H), 7.66 (d, J = 2.2 , 2H), 7.90 (d, J = 8.8, 2H). ¹³C NMR (CD₃OD): δ 109.6 (4C), 118.8 (2C), 119.5 (2C), 122.4 (2C), 129.0 (2C), 132.2 (2C), 139.7 (2C), 145.7 (4C), 149.3 (2C), 166.1 (2C). MS: M - H = 462.7.

### Example 14. 1,5-bis[(3,4,5-trihydroxybenzoyl)oxy]naphthalene (m)

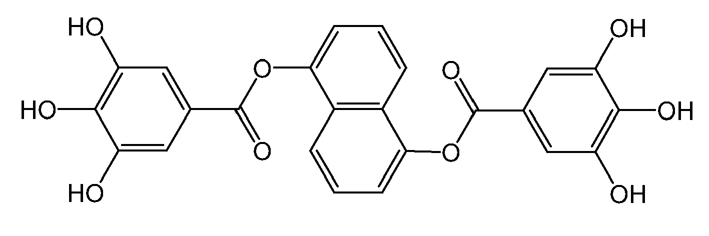

The title compound was prepared following general procedure described in example 1, starting from gallic acid and naphthalene-1,5-diol as starting materials.

Yield: 42% (a), 30% (b); mp: 265 °C (d). IR (KBr): 3406, 1718, 1618, 1209 cm⁻¹. ¹H NMR (CD₃OD): δ 7.29 (s, 4H), 7.33 (d, J = 7.0, 2H), 7.47 (t, J =7.6, 2H), 7.60 (d, J = 8.0, 2H). ¹³C NMR (DMSO-*d*₆): δ 109.2 (4C), 117.5 (2C), 119.1 (2C), 119.3 (2C), 127.8 (2C), 128.4 (2C), 139.6 (2C), 145.8 (4C), 146.7 (2C), 164.5 (2C). MS: M - H = 462.7.

### Example 15. 2,3-bis[(3,4,5-trihydroxybenzoyl)oxy]-1,1,4,4-tetramethyl-1,2,3,4-tetrahydronaphthalene (n)

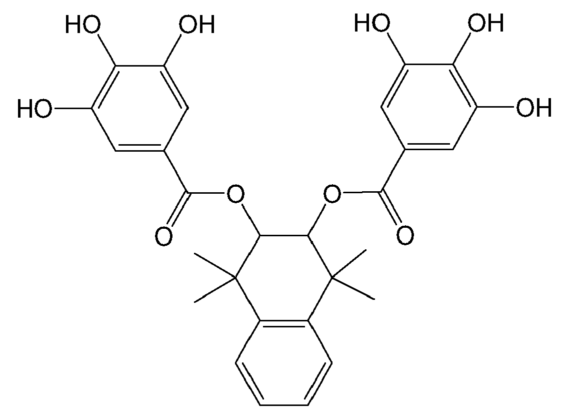

The title compound was prepared following general procedure described in example 1, starting from gallic acid and 1,1,4,4-tetramethyl-1,2,3,4-tetrahydronaphthalene-2,3-diol as starting materials.

Yield: 20% (a), 95% (b). ¹H NMR (CD₃OD): δ 1.40 (s, 6H), 1.52 (s, 6H), 5.61 (s, 2H), 6.99 (s, 4H), 7.24-7.27 (m, 2H), 7.43-7.46 (m, 2H). ¹³C NMR (CD₃OD): δ 27.5 (2C), 30.9 (2C), 40.3 (2C), 78.0 (2C), 110.4 (4C), 121.5 (2C), 127.7 (2C), 127.9 (2C), 140.0 (2C), 143.6 (2C), 146.6 (4C), 168.1 (2C).

### Example 16. 2,5-bis[(3,4,5-trihydroxybenzoyl)oxy]-1,1'-biphenyl (o)

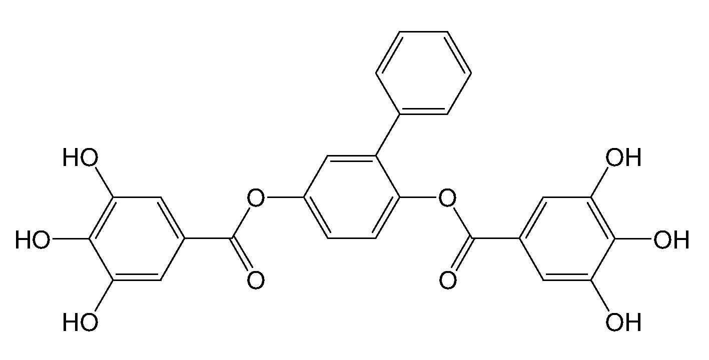

The title compound was prepared following general procedure described in example 1, starting from gallic acid and 1,1'-biphenyl-2,5-diol as starting materials.

Yield: 57% (a), 75% (b); mp: 114-116 °C. IR (KBr): 3362, 1701, 1616, 1165 cm⁻¹. ¹H NMR (CD₃OD): δ 7.06 (s, 2H), 7,24 (s, 2H), 7.26-7.38 (m, 6H), 7.45-7.49 (m, 2H). ¹³C NMR (CD₃OD): δ 110.6 (2C), 110.7 (2C), 120.4, 120.5, 122.8 (2C), 124.9; 125.4; 128.9; 129.5 (2C), 130.0 (2C), 137.6; 138.2, 140.7; 146.6 (2C), 146.8 (2C), 147.0, 150.4, 166.9, 167.0. MS: M - H = 488.6.

### Example 17. 6-(benzoyloxy)-1,1'-biphenyl-3-yl 3,4,5-trihydroxybenzoate (p)

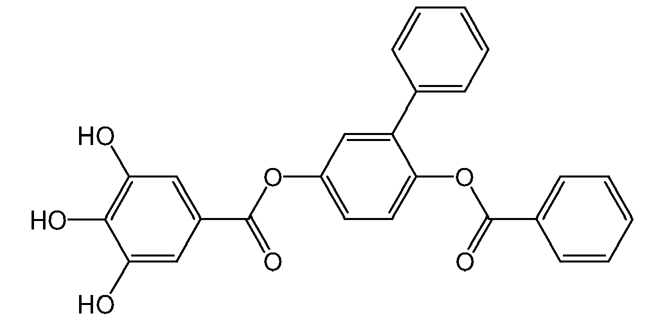

The title compound was prepared following general procedure described in example 1, starting from gallic acid, benzoic acid and 1,1'-biphenyl-2,5-diol as starting materials.

Yield: 20% (a), 54% (b); mp: 82-84 °C. IR (KBr): 3381, 1713, 1611, 1164 cm⁻¹. ¹H NMR (CD₃OD): δ 7.07 (s, 2H), 7.29-7.39 (m, 6H), 7.47-7.50 (m, 2H), 7.55-7.60 (m, 2H), 7.68-7.74 (m, 1H), 8.20-8.24 (m, 2H). ¹³C NMR (CD₃OD): δ 110.6 (2C), 120.2, 122.8, 124.8, 125.4, 128.9, 129.4 (2C), 129.8 (2C), 129.9 (2C), 130.6, 131.1, (2C), 135.0, 137.6, 138.0, 140.5, 146.6 (2C), 147.1, 150.0, 166.6, 166.9. MS: M + Na = 464.9.

### Example 18. 4,4'-bis[(3,4,5-trihydroxybenzoyl)oxy]-1,1'-biphenyl (q)

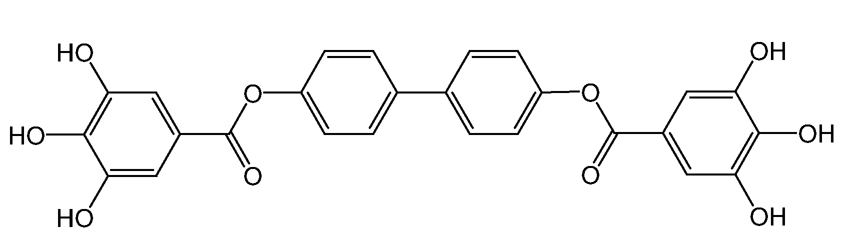

The title compound was prepared following general procedure described in example 1, starting from gallic acid and 1,1'-biphenyl-4,4'-diol as starting materials.

Yield: 44% (a), 70% (b); mp: 268 °C (d). IR (KBr): 3396, 1701, 1608, 1192 cm⁻¹. ¹H NMR (CD₃OD): δ 7.24 (s, 4H), 7.24-7.28 (m, 4H), 7.67-7.71 (m, 4H). ¹³_{C} NMR (CD₃OD): δ 110.8 (4C), 120.8 (2C), 123.5 (4C), 129.2 (4C), 139.4 (2C), 140.6 (2C), 146.9 (4C), 152.4 (2C), 167.2 (2C). MS: M - H = 488.8.

### Example 19. 4,4'-bis[(2,6-dihydroxyisonicotinoyl)oxy]-1,1'-biphenyl (r)

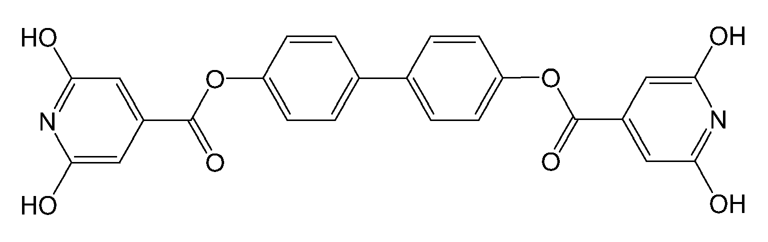

The title compound was prepared following general procedure described in example 1, starting from 2,6-dihydroxyisonicotinic acid and 1,1'-biphenyl-4,4'-diol as starting materials.

Yield: 26% (a), 66% (b); mp: 285 °C (d). IR (KBr): 3452, 1742, 1645, 1234 cm⁻¹. ¹H NMR (CD₃OD): δ 6.55 (s, 4H), 7.47 (d, *J* = 8.6, 4H), 7.87 (d, *J* = 8.6, 4H). MS: M - H = 458.7.

### Example 20. Cytotoxicity assays

MCF-7 and MDA-MB-231 breast cancer cells were obtained from the American Type Culture Collection (ATCC) and were routinely grown in Dulbecco's modified Eagle's medium (DMEM, Gibco) containing 10% fetal bovine serum (FBS, Bio-Whittaker), 1% L-glutamine, 1% sodium pyruvate, 50 U/mL penicillin and 50 µg/mL streptomycin. SK-Br3 breast cancer cells were obtained from Eucellbank (Universidad de Barcelona, Spain) and were passaged in McCoy's 5A medium containing 10% FBS, 1% L-glutamine, 1% sodium pyruvate, 50 U/mL penicillin and 50 µg/mL streptomycin. Non-malignant fibroblasts N-1 were obtained from Eucellbank (Universidad de Barcelona, Spain) and were passaged in DMEM medium containing 10% FBS, 1% L-glutamine, 1% sodium pyruvate, 50 U/mL penicillin and 50 µg/mL streptomycin. The cells remained free of *Mycoplasma* and were propagated in adherent culture according to established protocols. Cells were maintained at 37 °C in a humidified atmosphere of 95% air and 5% CO₂.

Drug sensitivity was determined using a standard colorimetric MTT (3-4,5-dimethylthiazol-2-yl-2,5-diphenyltetrazolium bromide) assay. Briefly, cells were plated out at a density of 7 × 10³ cells/100 µL/well in 96-well microtitre plates and allowed an overnight period for attachment. Then the medium was removed and fresh medium along with various concentrations of the tested compound were added to the cultures for a period of 48 h. Following the treatment, the cells were fed with drug-free medium (100 µL/well) and 10 µL of MTT solution (5 mg/mL; Sigma, St. Louis, MO), and the incubation was prolonged for 3 h at 37 °C. After carefully removing the supernatants, the MTT-formazan crystals formed by metabolically viable cells were dissolved in dimethyl sulphoxide (100 µL/well) and the absorbance was measured at 570 nm in a multi-well plate reader (Model Anthos Labtec 2010 1.7 reader).

The results obtained from these cytotoxicity assays are presented below in Table 1.

**Table 1. Growth inhibition of novel polyhydroxyderivatives (I) against human breast cancer cells**

| **compounds** | **SK-Br3** | **MCF-7** | **MDA-MB-231** |
|---|---|---|---|
| **EGCG** | 149 ± 19.8 | 205 ± 7.1 | 196.7 ± 15.3 |
| **a** | 125.5±5.2 | 165±8.2 | 163±5.2 |
| **b** | 42±7.1 | 162.4±17.8 | 172.8±40.2 |
| **c** | 23.3±2.1 | 4.5±14.9 | 54.7±7.6 |
| **d** | 8.3±1.1 | 30±0.1 | 28±1 |
| **e** | 76.5±9.2 | 89±28.7 | 136±26.9 |
| **f** | 21.3±6.7 | 46.3±18 | 78.7 ± 4 |
| **g** | 107.3 ± 29.7 | N.D. | 121 ±5.7 |
| **h** | 20.7±11.5 | N.D. | 19±1.1 |
| **i** | 28.7±0.3 | 44.4±16.3 | 62.5±10.6 |
| **j** | 115±7.1 | N.D. | 24±0.9 |
| **k** | 37.5±6.4 | N.D. | 27±11.3 |
| **o** | 17.5±2.6 | 10±2.8 | 40±2.8 |
| **p** | 73.5 ± 4.9 | N.D. | > 150 |
| **q** | 4.4 ±1.4 | 2.5 ± 0.1 | 5.2 ± 3.5 |
| **DMSO control** | N.T. | N.T. | N.T. |

IC₅₀ values are the concentrations at which 50% of cell growth is inhibited; N.T.: no toxicity identified; N.D.: non determined.

### Example 21. Fatty acid synthase activity assay

FASN activity of compounds **a, b, e, f,** and i was assayed in particle-free supernatants by recording spectrophotometrically at 37 °C (Lambda Bio 20, Perkin Elmer, EUA; using UV Kinlab 2.80.02 software) the decrease of A₃₄₀ₙₘ due to the oxidation of NADPH, as we previously described. After 6, 12, 24 h of exposure to the tested compound, the cells were harvested by treatment with trypsin-EDTA solution, pelleted by centrifugation, washed twice and resuspended in cold PBS. The cells were sonicated during 30 min at 4 °C (PSelecta ultrasons) and centrifuged for 15 min at 4 °C to keep supernatants particle-free. A sample was taken to measure the protein content by a BioRad assay (Bio-Rad Laboratories). One-hundred and twenty micrograms of protein were pre-incubated during 15 min at 37 °C in 0.2 M of potassium phosphate buffer, pH 7.0, for temperature equilibration. The sample was then added to the reaction mixture: 200 mM potassium phosphate buffer, pH 7.0, 1 mM EDTA (Sigma), 1 mM dithiothreitol (Sigma), 30 µM acetyl-CoA (Sigma), 0.24 mM NADPH (Sigma), in 0.3 mL reaction volume were monitored at 340 nm for 3 min to measure the background NADPH oxidation. After the addition of 50 µM of malonyl-CoA (Sigma), the reaction was assayed for additional 10 min to determine FASN-dependent oxidation of NADPH. Rates were corrected for the background rate of NADPH oxidation in the presence of acetyl-CoA. FASN activity was expressed in nmol NADPH oxidized min⁻¹ mg protein⁻¹.

After cancer cell exposure to EGCG and compounds **a, e, f** and i, FASN activity decreased in a time-dependent manner until 24 hours (Fig. 1). Exposure of SK-Br3 cells to compounds **f** and **i** for 24 h further reduced FASN activity to 10 ± 4 % and 31 ± 16 % of control cells (Fig. 1). The decrease of FASN was not caused by changes in FASN protein levels, Western blot analysis revealed that treated and control SK-Br3 lysates exhibited an equal single band (~250 KDa) recognized by a rabbit monoclonal anti-FASN antibody.

Taken together, compounds f and i have marked cytotoxic effects on SK-Br3 breast cancer cells (IC₅₀ = 21.3 ± 6.7 µM and 28.7 ± 0.3 µM respectively, compared to IC₅₀ of EGCG 149.8 ± 19.8 µM), and also displayed the greatest inhibition of FASN activity (reduction of 50% of FASN activity after 6 h of cell exposure, Fig. 1). In addition, normal fibroblasts were not affected by compounds f and i at concentrations similar to IC₅₀. In summary, a striking correlation was observed between the lipogenesis inhibitory effect of compounds f and i and their cytotoxic effect on cancer cells.

### Example 22. Carnitine palmitoyltransferase 1 (CPT-1A) assay

The expression plasmid pRCPT-Iβ/pHW010 (provided by Gebre Woldegiorgis, Beaverton, Oregon. Published in Woldegiorgis G. et al. Biochem. Biophys. Res. Commun. 2004, 325, 660-4.) was linearized in the GAP gene promoter by digestion with Avrll and integrated into the GAPp locus of *P.pastoris* GS115 by electroporation. Histidine prototrophic transformants were selected on YND plates and grown on YND medium. Mitochondria were isolated by disrupting the yeast cells with glass beads as previously described.

CPT activity was assayed by the forward exchange method using L-[³H] carnitine as previously described (Nicot, C. et al., Biochem. Biophys. Res. Comm. 2004, 325, 2745-7). In a total volume of 0.5 mL, the standard enzyme assay mixture contained 0.2 mM L-[³H]carnitine (-5000 dpm/nmol), 80 µM palmitoyl-CoA, 20 mM HEPES (pH 7.0), 1 % fatty acid-free albumin, and 40-75 mM KCI, with or without malonyl-CoA as indicated. Reactions were initiated by addition of isolated intact yeast mitochondria. The reaction was linear up to 4 min, and all incubations were done at 30 °C for 3 min. Reactions were stopped by addition of 6% perchloric acid and were then centrifuged at 2000 rpm for 7 min. The resulting pellet was suspended in water, and the product [³H]palmitoylcarnitine was extracted with butanol at low pH. After centrifugation at 2000 rpm for 2 min, an aliquot of the butanol phase was transferred to a vial for radioactive counting.

As shown in Fig. 2 recombinant rat CPT-1A.. was significativally activated by reference compound C75 (up to 129 ± 6 %) and, interestingly, compound f (89 ± 4 %) and compound i (90 ± 5 %) did not stimulate CPT-1A activity.

### Example 23. Immunoblot analysis of FASN, p185^{HER2/neu}, phospo-p185^{HER2/neu}, anti-ERK1/2, anti-phospo-ERK1/2, anti-AKT, anti-phospo-AKT^{Ser473} and PARP.

Following the treatment of SK-Br3 cells with the studied compound at the desired concentrations and time intervals, cells were harvested by treatment with trypsin-EDTA solution, washed twice with PBS and stored at - 80°C. The cells were lysed in lysis buffer (1 mM EDTA, 150 mM NaCl, 100 µg/mL PMSF and 50 mM Tris-HCl, pH 7.5) and kept at 4 °C while they were routinely mixed every 2 min on the vortex during 30 min. A sample was taken for measurement of protein content by a BioRad assay (Bio-Rad Laboratories). Equal amounts of protein were heated in sodium dodecyl sulphate (SDS) sample buffer (Laemmli) for 5 min at 95 °C, separated on a 3-8% SDS-polyacrylamide gel (FASN, p185^{HER2/neu}, phospo-p185^{HER2/neu}) or 4-12% SDS-polyacrylamide gel (AKT, phospho-AKT, ERK1/2, phospo-ERK1/2 and PARP) and transferred onto nitrocellulose membranes. The membranes were incubated for 1 h at room temperature in blocking buffer (2.5% powdered-skim milk in TBS-T [10 mM Tris-HCl pH 8.0, 150 mM NaCl and 0.05% Tween-20]) to prevent non-specific antibody binding. The primary antibody used was a monoclonal antibody. Antibody dilution was prepared in blocking solution and blots were incubated with monoclonal antibody overnight at 4 °C. After 3x5 min washing in TBS-T, blots were incubated for 1 h with anti-mouse IgG peroxidase conjugate and revealed employing a commercial kit (West Pico chemiluminescent substrate). Blots were re-probed with an antibody for β-actin to control for protein loading and transfer.

Western blot analysis for poly-ADP-ribose polymerase (PARP) was performed on SK-Br3 cells exposed for 12, 24 and 48 h to compound f (30 µM) and compound **i** (30 µM). Apoptosis and induction of caspase activity was confirmed by Western blotting analysis showing cleavage of PARP. Treatment with compounds f and i resulted in a marked increase on 89 KDa, product of PARP cleavage, in a time-dependent manner.

We previously reported that FASN inhibitors (C75 and EGCG) induced apoptosis and blocked the activation of the oncogene HER2 and their downstream signal transduction pathways ERK1/2 and P13/AKT, in SK-Br3 breast cancer cells (which are a model of over-expression of HER2). We have examined the time-dependent effects of compound **f** (30 µM) and compound **i** (30 µM) on HER2, AKT and ERK1/2 activation. We have observed a total decrease in the levels of p-HER2 proteins within 6 h after treatment with compounds **f** and i. In fact, the expression levels of p-HER2 proteins markedly decreased within 2 h after the exposure to compounds **f** and i. During this period, there was no significant change in the level of total HER2, as assessed by Western blotting analysis or by HER2-specific ELISA.

Activation of p-ERK1/2 proteins also displayed a marked decrease at 6 h after treatment with derivatives f and i. The expression levels of p-AKT proteins also showed a low decrease at 6 hours after the treatment with compounds f and i. Anyway, a marked decrease of p-AKT was observed 24 h after the treatment with compound f and 12 h after the treatment with compound **i.** During this period, there was no significant change in the total level of the respective proteins.

## Claims

1. A compound of formula (I), its stereoisomers, crystalline forms, prodrugs, pharmaceutically acceptable salts and pharmaceutically acceptable solvates: 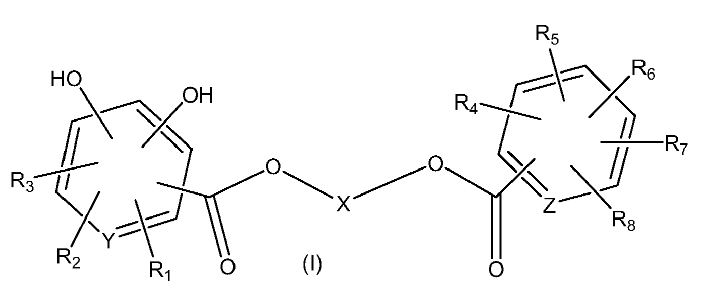 wherein:
R₁, R₂, R₃, R₄, R₅, R₆, R₇ and R₈ are independently selected from the group consisting of H, (C₁-C₄)-alkyl, (C₁-C₄)-alkoxy, halogen, nitro, NH₂, (C₁-C₄)alkyl ester, CONH₂, (C₁-C₄)alkylamide, CF₃, and hydroxyl;
Y and Z are each independently selected from C and N;
X is a biradical derived from an aromatic monoheterocycle being at least one of the members of the ring independently selected from N, O, and S; or a biradical derived from one of the known ring systems containing 2-3 rings of 5-6 members each ring, being the rings aromatic or partially unsaturated, isolated or fused; each ring optionally substituted with one or more groups selected from the group consisting of (C₁-C₄)-alkyl, and (C₁-C₄)-alkoxy, halogen, nitro, NH₂, (C₁-C₄)alkyl ester, CONH₂ and (C₁-C₄)alkylamide; being each one of the members of the ring independently selected from C, N, O and S;
with the proviso that X is not chromane, anthracene or a biradical of formula:

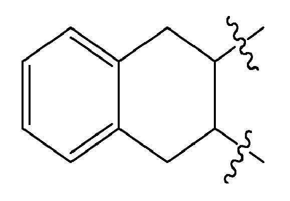

2. The compound according to claim 1, wherein X is a biradical derived from an aromatic monoheterocycle being at least one of the members of the ring independently selected from N, O, and S; or a biradical derived from one of the known ring systems containing 2-3 rings of 5-6 members each ring, being the rings aromatic or partially unsaturated, isolated or fused; each optionally substituted with one or more groups selected from the group consisting of (C₁-C₄)-alkyl, and (C₁-C₄)-alkoxy, halogen, nitro, NH₂, (C₁-C₄) alkyl ester, CONH₂ and (C₁-C₄)alkylamide; being each one of the members of the ring independently selected from C and N with the proviso that X is not anthracene or a biradical of formula

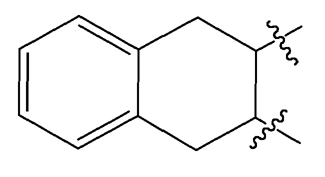

3. The compound according to any of the claims 1-2, wherein X is selected from the group consisting of naphthalene, pyridine, and biphenyl optionally substituted by (C₁-C₄)-alkyl, nitro, halogen, and hydroxyl.

4. The compound according to any of the claims 1-3, wherein X is selected from the group consisting of

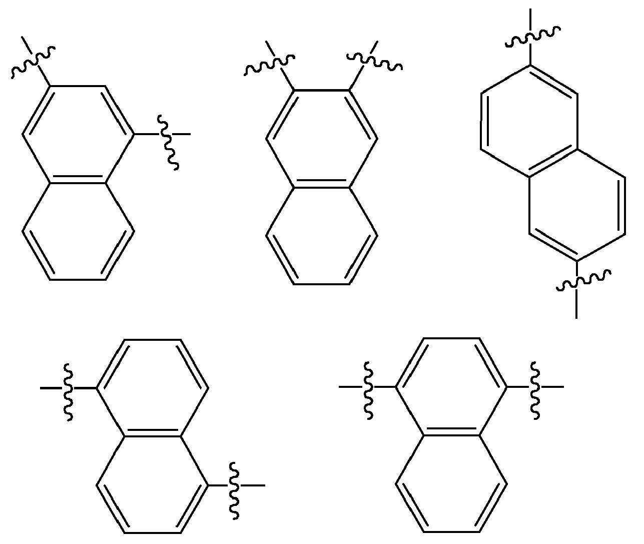

5. The compound according to any of the claims 1-4, wherein at least two of R₄, R₅, R₆, R₇ and R₈ are hydroxyl.

6. The compound according to any of the claims 1-5, which is selected from the following:
2,3-bis[(3,4,5-trihydroxybenzoyl)oxy]naphthalene (e);
1,3-bis[(3,4,5-trihydroxybenzoyl)oxy]naphthalene (f);
1,3-bis[(3,5-dihydroxybenzoyl)oxy]naphthalene (g);
1,3-bis[(3,4-dihydroxybenzoyl)oxy]naphthalene (h);
1,4-bis[(3,4,5-trihydroxybenzoyl)oxy]naphthalene (i);
1,4-bis[(3,5-dihydroxybenzoyl)oxy]naphthalene (j);
1,4-bis[(3,4-dihydroxybenzoyl)oxy]naphthalene (k);
2,6-bis[(3,4,5-trihydroxybenzoyl)oxy]naphthalene (l);
1,5-bis[(3,4,5-trihydroxybenzoyl)oxy]naphthalene (m);
2,5-bis[(3,4,5-trihydroxybenzoyl)oxy]-1,1'-biphenyl (o);
6-(benzoyloxy)-1,1'-biphenyl-3-yl 3,4,5-trihydroxybenzoate (p);
4,4'-bis[(3,4,5-trihydroxybenzoyl)oxy]-1,1'-biphenyl (q); and
4,4'-bis[(2,6-dihydroxyisonicotinoyl)oxy]-1,1'-biphenyl (r).

7. The compound according to claim 6, which is selected from the following:
1,3-bis[(3,4,5-trihydroxybenzoyl)oxy]naphthalene (f);
1,3-bis[(3,4-dihydroxybenzoyl)oxy]naphthalene (h);
1,4-bis[(3,4,5-trihydroxybenzoyl)oxy]naphthalene (i);
2,5-bis[(3,4,5-trihydroxybenzoyl)oxy]-1,1'-biphenyl (o); and
4,4'-bis[(3,4,5-trihydroxybenzoyl)oxy]-1,1'-biphenyl (q).

8. A pharmaceutical composition comprising an effective amount of a compound of formula (I), as defined in claim 1, its stereoisomers, crystalline forms, prodrugs, pharmaceutically acceptable salts and pharmaceutically acceptable solvates, with the proviso that X is not chromane or 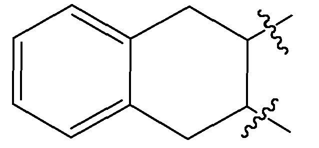 in combination with appropriate amounts of pharmaceutically acceptable diluents or carriers.

9. Use of a compound of formula **(I),** as defined in claim 1, its stereoisomers, crystalline forms, prodrugs, pharmaceutically acceptable salts and pharmaceutically acceptable solvates, with the proviso that X is not chromane or 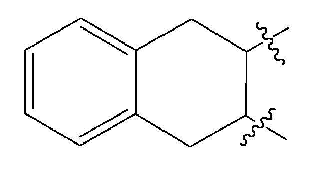 for the manufacture of a medicament for the prophylaxis and/or treatment of a disorder mediated by FASN and associated clinical symptoms in a human person.

10. Use according to claim 9, wherein the disorder is selected from cancer and obesity.

11. Use according to claim 10, wherein the cancer is selected from the group consisting of autoimmune lymphoproliferative syndrome (ALPS), chronic lymphoblastic leukemia, hairy cell leukemia, chronic lymphatic leukemia, peripheral T-cell lymphoma, small lymphocytic lymphoma, mantle cell lymphoma, follicular lymphoma, Burkitt's lymphoma, Epstein-Barr virus-positive T cell lymphoma, histiocytic lymphoma, Hodgkin's disease, diffuse aggressive lymphoma, acute lymphatic leukemia, T gamma lymphoproliferative disease, cutaneous B cell lymphoma, cutaneous T cell lymphoma, Sezary syndrome, acute myelogenous leukemia, chronic or acute lymphoblastic leukemia, hairy cell leukemia, sarcoma, osteosarcoma, breast cancer, squamous cell carcinoma, Epstein-Barr virus-positive, nasopharyngeal carcinoma, glioma, colon, stomach, prostate, renal cell, cervical and ovarian cancers, lung cancer, including small cell lung carcinoma, and non-small cell lung carcinoma.

12. Use according to claim 11, wherein the cancer is selected from the group consisting of breast, prostate, colon, ovary, endometrium, mesothelium, lung, thyroid, stomach and brain cancer.

13. Use according to claim 9, wherein symptoms associated with cancer are selected from cancer-associated cachexia, fatigue, asthenia, paraneoplastic syndrome of cachexia and hypercalcemia.

14. Use of a compound of formula (I), as defined in claim 1, its stereoisomers, crystalline forms, prodrugs, pharmaceutically acceptable salts and pharmaceutically acceptable solvates thereof for the preparation of a medicament for the treatment and/or prophylaxis of a disorder mediated by FASN and associated clinical symptoms

15. A process for the preparation of the compounds of formula (I), as defined in claim 1, with the proviso that X is not chromane, anthracene or a biradical of formula 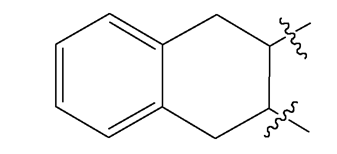 which comprises the following steps:
a) reacting a dihydroxyderivative of formula (**II**) 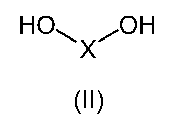 wherein X is as defined in claim 1; with a carboxylic acid of formula **(III)** and with a carboxylic acid of formula **(IV)** 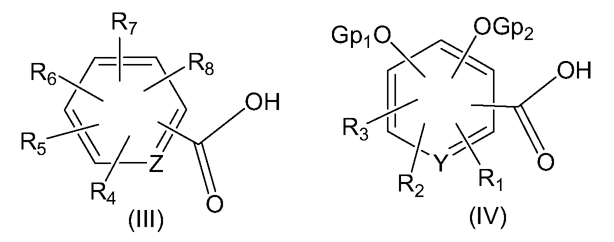 wherein Y, Z and R₁-R₈ are as defined in claim 1, and Gp₁ and Gp₂ are each independently a hydroxyl protecting group; to give a compound of formula (V) wherein Gp₁, Gp₂, R₁-R₈ are as defined in claim 1; 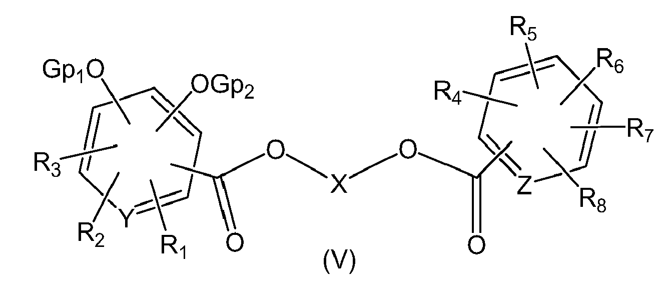
b) deprotecting the compound of formula (V) to remove the protecting groups; and
c) optionally converting a compound of formula (I) into its pharmaceutically acceptable salt; and/or separating a mixture of isomers of a compound of formula (I) to isolate one of such isomers substantially free from the other isomer; and/or transforming it into a prodrug thereof.

16. A kit comprising separate containers containing a pharmaceutical composition according to claim 8, and a reconstituting agent and instructions for the use of each actor in combination for the treatment or prevention of cancer.
